# EUROPEAN PATENT APPLICATION

(11) **EP 2 561 879 A1**
(43) Date of publication of application: **27.02.2013**
(21) Application number: 11178511.9
(22) Date of filing: 23.08.2011
(51) Int. Cl.: A61K 35/407, A61K 38/19, A61K 38/20, A61K 38/21, A61K 31/714, A61P 31/12, A61P 43/00

(54) **Macrophage activating factor for use in the treatment of chronic fatigue syndrome (CFS) and CFS-related diseases and disorders**

(71) Applicant: Protea Biopharma N.V., 1120 Neder-Over-Heembeek (BE)
(72) Inventor: Roelant, Christiaan, B-3000 Leuven (BE); De Meirleir, Kenny, B-2800 Mechelen (BE)
(74) Representative: Paemen, Liesbet R.J.

(57) **Abstract**

The present invention relates to Macrophage Activating Factors such as GcMAF and compositions thereof, for use in the treatment of a patient suffering from CFS/ME and/or XMRV infection.

## Description

### FIELD OF THE INVENTION

The present invention relates to the therapeutic and/or prophylactic use of Macrophage Activating Factors.

### BACKGROUND OF THE INVENTION

Macrophage Activating Factors (MAFs) are lymphokines that prime macrophages to become cytotoxic to tumors. These factors also control the expression of la antigens on the macrophage cell surface. There are indications that the use of MAFs, particularly the GcMAF protein, is effective in the treatment of certain cancers and HIV (EP0837932, Yamamoto).
Chronic fatigue syndrome (CFS) is a difficult to diagnose, ubiquitous disorder characterized by extreme fatigue, lymph gland enlargement and constitutional symptoms such as weight loss, loss of appetite, memory deterioration and loss of intelligence in some patients. Some CFS patients manifest neuropsychiatric changes such as depression, loss of memory and similar derangements. Thus, chronic fatigue syndrome is sometimes difficult to distinguish from entirely neurological disorders, particularly situational depression. An accumulating body of evidence suggests that CFS is associated with disregulation of both humoral and cellular immunity, including mitogen response, reactivation of viruses, abnormal cytokine production, diminished natural killer cell function and changes in intermediary metabolites. CFS is also referred to by some as CFS/ME (myalgic encephalomyelitis). The term myalgic encephalomyelitis refers to muscle aches or pains and inflammation of the brain and spinal cord. While inflammation of the brain is not observed in all CFS patients, the group of patients referred to under CFS/ME typically suffer from the same symptoms and lack a clear etiology

A number of further diseases have been associated with CFS/ME because of the similarity of the symptoms observed with CFS/ME patients and patients suffering from such CFS/ME-related diseases. For instance, fibromyalgia, autoimmune thyroid disease, xenotropic murine leukemia virus-related virus (XMRV) and several other diseases have been suggested as being related to CFS/ME and present similar symptoms of widespread pain, fatigue, feeling run down, sluggish muscle cramps and pains, unexplained or excessive weight gain, inability to lose weight, gastrointestinal problems, irritable bowel syndrome, poor sleeping, headaches and migraines, constipation and exhaustion.

For many CFS/ME patients, cognitive behavioural therapy and graded exercise therapy have shown only moderate effectiveness (Whiting et al. JAMA 286: 1360-1368, 2001). Many patients do not fully recover from CFS/ME even with treatment (Rimes et al. Occupational Medicine 55: 32-39, 2005). Other treatments of CFS/ME have been proposed but their effectiveness has not been confirmed (Prins et al. Lancet 367: 346-355, 2006).
XMRV is an infectious human gamma-retrovirus, which has been linked to both CFS/ME and human prostate cancer (Kearney et al. JID 202: 1463-1466, 2011). Although *in vitro* studies have indicated that raltegravir (Trade name Isentress^{®}) can act as an inhibitor for XMRV replication (Singh et al., PLoS ONE 5: e9948, 2010), its effect on the *in vivo* XMRV replication is not yet documented.

There remains a need for compounds for use in the treatment and/or prevention of CFS/ME and CFS/ME-related diseases, such as XMRV infection. More particularly, there is a need for novel and improved compounds that are capable of reducing, inhibiting or decreasing the particular symptoms that are observed in patients suffering from CFS/ME and/or CFS/ME-related diseases and disorders, such as for example XMRV infection.

### SUMMARY OF THE INVENTION

The present invention relates to a Macrophage Activating Factor (MAF) for use in the treatment of a patient suffering from chronic fatigue syndrome/myalgic encephalomyelitis (CFS/ME) and/or one or more CFS/ME-related diseases and/or disorders, such as infection by xenotropic murine leukemia virus-related virus (XMRV). In particular embodiments, the present invention provides a composition comprising a MAF for use in the treatment of a patient suffering from CFS/ME and/or an infection by XMRV. In a further aspect the present invention provides use of a MAF for the manufacture of a medicament for the treatment of CFS/ME and/or CFS/ME related diseases and disorders, such as infection by XMRV in a mammal.

Indeed, the inventors found that the administration of a MAF leads to a significant decrease of the manifestation of the symptoms of CFS/ME and CFS/ME-related diseases, more particularly of XMRV. For instance, the inventors have found that administration with a MAF, more particularly GcMAF, is capable of suppressing or reducing at least one of the biological parameters associated with CFS/ME and CFS/ME-related diseases. More particularly, it has been established that indicators of Natural Killer (NK) cell activity and inflammation, are affected when patients suffering from such diseases are treated with MAF. Thus, in particular embodiments, the patient is characterized by reduced NK cell activity and increased inflammatory mediators. In certain embodiments, the patient is diagnosed with CFS/ME and with XMRV infection.
It is further considered that treatment with MAF ensures important clinical improvement, for example as measured on the Karnofsky scale or the Bell disability scale (D.S. Bell, The Doctor's Guide to Chronic Fatigue Syndrome, Reading, MA: Addison-Wesley, 1994). Specifically, the MAF or compositions according to the present invention are useful for reducing at least one symptom chosen from the group consisting of substantial impairment in short-term memory or concentration, sore throat, tender lymph nodes, muscle pain, multi-joint pain without swelling or redness, headaches of a new type, pattern, or severity; unrefreshing sleep, post-exertional malaise lasting more than 24 hours, widespread pain, fatigue, feeling run down, sluggish muscle cramps and pains, unexplained or excessive weight gain, inability to lose weight, gastrointestinal problems, irritable bowel syndrome, poor sleeping, headaches and migraines, constipation, orthostatism and exhaustion.

In particular embodiments, the present invention provides a MAF as described above provided in a conjugate or in a pharmaceutical composition comprising a pharmaceutically acceptable excipient and a therapeutically effective amount of said MAF.
In certain embodiments, the MAF as described herein is the only active ingredient of the composition as described herein. In further embodiments, the present invention provides a MAF as described above provided in a conjugate or in a pharmaceutical composition consisting of a pharmaceutically acceptable excipient and a therapeutically effective amount of said MAF.

In particular embodiments, the present invention relates to methods for treating a patient having been diagnosed with CFS/ME and/or one or more CFS/ME-related disease(s) based on aberrant ecto-nox functioning as described herein. In particular embodiments, the patient described above is diagnosed with aberrant and/or increased metabolite production.

In particular embodiments, the MAF in the present invention is GcMAF protein.

A further aspect of the present invention relates to the compositions as described above, wherein said MAF according to the invention is provided in combination with one or more other pharmacologically active compounds or ingredients. In particular embodiments, one of the one or more other active ingredients is Vitamin B12. In certain embodiments, at least one of the one or more other active ingredients is selected from the group consisting of interferon alpha, interferon beta, interferon gamma and interleukin-7. In certain embodiments, at least one of the one or more other active ingredients is selected from a viral inhibitor and/or an ecto-nox-modifying agent. In certain embodiments, the compositions according to the present invention further comprise a mammalian liver extract.

In particular embodiments, the present invention relates to compositions as described above, for enteral administration. In another embodiment, the present invention relates to compositions as described above, for parenteral administration.

### DETAILED DESCRIPTION OF THE INVENTION

In the following passages, different aspects of the invention are described in more detail. Each aspect so described may be combined with any other aspect or aspects unless clearly indicated to the contrary. In particular, any feature indicated as being preferred or advantageous may be combined with any other feature or features indicated as being preferred or advantageous.

In the context of the present invention, the terms used are to be construed in accordance with the following definitions, unless a context dictates otherwise.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

This invention relates to the use of Macrophage Activating Factors ("MAFs") in the treatment of CFS/ME and/or in the treatment of infection by xenotropic murine leukemia virus-related virus (XMRV) in a mammal.

The term "Macrophage activating factor" or "MAF" as referred to herein designates a lymphokine protein that stimulates monocytes and/or macrophages (designated as macrophages hereafter). This stimulation results in the macrophages to become cytotoxic to tumors, and/or primes the phagocytosis of pathogens by macrophages.
In particular, the present invention relates to a GcMAF protein for use in the treatment of a mammal suffering from CFS/ME and/or one or more CFS/ME-related diseases, such as for example infection by XMRV.
The term "GcMAF" as referred to herein designates a Gc (group specific component) protein derived macrophage activating protein, or a domain III (natural or cloned) derived macrophage activating protein (e.g. US patent 6410269, Yamamoto, which is included herein by reference). The term "domain III" as referred to herein designates the domain III region of the Gc protein. The term "Gc protein" as referred to herein designates a Vitamin D binding protein (DBP); DBP is also known as gc-globulin.

A first aspect of the invention relates to a composition comprising a Macrophage Activating Factor (MAF) for use in the treatment of patients diagnosed with chronic fatigue syndrome/myalgic encephalomyelitis (CFS/ME) and/or CFS/ME-related diseases and disorders. Accordingly, the present invention provides for a treatment that reduces, inhibits or decreases the symptoms of CFS/ME and CFS/ME-related diseases, such as XMRV infection.
It is known that CFS/ME and CFS/ME related diseases seriously interfere with the immune system, causing a dysfunctioning thereof. The present inventors have found that by using a composition comprising a MAF, the activity of natural killer cells (or NK cells), i.e. cytotoxic lymphocytes that play a major role in the regulation of the innate immune system, is increased. Thus, use of MAF in patients suffering from CFS/ME or CFS/ME-related diseases causes a positive stimulation of the immune system. Furthermore, the inventors have found that by using a MAF in the treatment of patients suffering from CFS/ME and/or CFS/ME-related diseases, inflammation is reduced.

The term "chronic fatigue syndrome" or "CFS" as referred to herein designates a condition which is diagnosed based on the following criteria (as developed by the U.S. Centers for Disease Control and Prevention in 1994):
1. Clinically evaluated, unexplained persistent or relapsing chronic fatigue that is of new or definite onset (i.e., not lifelong), is not the result of ongoing exertion, is not substantially alleviated by rest, and results in substantial reduction in previous levels of occupational, educational, social, or personal activities.
2. The concurrent occurrence of four or more of the following symptoms: substantial impairment in short-term memory or concentration; sore throat; tender lymph nodes; muscle pain; multi-joint pain without swelling or redness; headaches of a new type, pattern, or severity; unrefreshing sleep; and post-exertional malaise lasting more than 24 hours. These symptoms must have persisted or recurred during 6 or more consecutive months of illness and must not have predated the fatigue.

The term "CFS/ME and CFS/ME-related diseases and disorders" refers to diseases and/or disorders with having an underlying biological mechanism of action and/or signaling pathways that are the same or similar to those known to underlie CFS and/or presenting the same or similar symptoms as observed with patients suffering from CFS.

Several standardized tests exist to follow up patients suffering from CFS/ME, such as the Activities of Daily Living Questionnaire (ADL)(see e.g. Collin et al. Int. Disabil. Stud. 10:61-63, 1988), the (Cognitive Deficit Subset of the) Symptom Checklist Questionnaire (SCL-90-R) (see e.g. Diaz-Mitoma et al. Journal of Chronic Fatigue Syndrome 11:71-95, 2003) and the Karnofsky Performance Score (KPS) (Karnofsky D and Burchenal JH. Clinical evaluation of chemotherapeutic agents in cancer. In Macleod CM. (ed) Evaluation of chemotherapeutic agents. Columbia University Press, New York 1949; 199-205). An alternative performance score specifically developed for CFS/ME patients is the Bell disability scale (D.S. Bell, The Doctor's Guide to Chronic Fatigue Syndrome, Reading, MA: Addison-Wesley, 1994).
Thus, in particular embodiments, the present invention provides for compositions comprising a MAF, for use in the treatment of CFS/ME and CFS/ME related diseases, which are capable of reducing the clinical symptoms of CFS/ME and/or CFS/ME related diseases as determined by one or more of the above-described evaluation methods. More particularly the compositions of the invention ensure a 10% improvement, more particularly a 20%, 30%, 40%, 50%, 60%, 70% or 80% or more improvement of clinical symptoms compared to prior to administration of the composition of the invention.

On a physiological level, CFS/ME has been associated with a variety of immune abnormalities. For instance, it has been reported that CFS/ME patients suffer from a suppression of NK cell activity (Whiteside et al. Am. J. Med. 105: 27S-34S, 1998). Published results also demonstrate an association between clinical improvement of patients suffering from CFS/ME and enhanced NK cell activity (Diaz-Mitoma et al. Journal of Chronic Fatigue Syndrome 11:71-95, 2003). It is also reported that CFS/ME is associated with diminished intracellular perforin, a protein found in the granules of NK cells (Maher et al., Clin. Exp. Immunol. 2002, 142, 505). Therefore, NK activity and/or intracellular perforin levels are suitable indicators for monitoring the disease state in patients suffering from CFS/ME.
Additionally, CFS/ME is associated with indications of inflammation. For example, CFS/ME patients often exhibit elevated levels of pro-inflammatory cytokines such as Interleukin-8 (IL-8). Patients suffering from CFS/ME also frequently have elevated C4a serum levels (Sorensen et al. J. Allergy Clin. Immunol. 112: 397-403, 2003). C4a is an anaphylatoxin generated by cleavage of complement component 4 (C4), upon activation of the complement system. Anaphylatoxins are able to trigger degranulation of endothelial cells, mast cells or phagocytes, which produce a local inflammatory response. Thus, indications of inflammation such as IL-8 and/or C4a levels are additional parameters which can be monitored in order to follow up patients suffering from CFS/ME.
In particular embodiments, the present invention provides for compositions comprising a MAF, for use in the treatment of CFS/ME and CFS/ME related diseases, which are capable of reducing the physiological symptoms of CFS/ME and/or CFS/ME related diseases such as but not limited to reduced NK cell activity and elevated inflammatory response. Most particularly, the invention provides compositions capable of ensuring a 10% improvement, more particularly a 20%, 30%, 40%, 50%, 60%, 70% or 80% or more improvement of the physiological symptoms of CFS/ME compared to prior to administration of the composition of the invention. More particularly, the compositions of the invention can be used to normalize the NK activity and/or inflammatory response in a patient suffering from CFS/ME or a CFS/ME related disorder.

It will be understood that in view of the nature of the disease and the diagnostic criteria, not all patients suffering from CFS/ME are characterized by reduced NK cell activity and/or increased inflammatory responses. Thus, in particular embodiments, the present invention provides compositions for use in the treatment of patients suffering from CFS/ME, wherein the patient is diagnosed with increased C4a serum level, increased IL-8 serum level and/or decreased NK cell activity. In particular embodiments, the patient is diagnosed with at least one element selected from the group consisting of increased C4a serum level, increased IL-8 serum level and decreased NK cell activity. "Increased C4a serum level" as used herein refers to a C4a serum level above 1500 ng/mL, preferably above 2500 ng/mL. "Increased IL-8 serum level" as used herein refers to an IL-8 serum level above 15 pg/mL, preferably above 25 pg/mL. "Decreased NK cell activity" as used herein refers to a perforin mRNA expression in peripheral blood mononuclear cells (PBMCs) below.

It has been described that further indicators of CFS/ME include an aberrant functioning of constitutive ecto-nox proteins. WO2010109009, which is hereby incorporated by reference, provides methods for determining the presence of aberrant ecto-nox functioning in the sample of a patient.

The term "ecto-nox protein" or "membrane NADH oxidase" as used herein refers to a cell-surface protein with both hydroquinone oxidase and protein-disulfide-thiol interchange activity. Constitutive ecto-nox proteins or prions are constitutively present in cellular membranes. They differ in this respect from mutated ecto-nox proteins present e.g. in cancer cells, such as t-nox.

More particularly the method described in WO2010109009 can be used to determine whether a patient is suffering from CFS/ME, more particularly whether the patient is susceptible to treatment with a compound capable of reducing aberrant ecto-nox functioning.

It is noted in this regard that the term "normal" when used in the context of ecto-nox protein or membrane NADH oxidase as used herein refers to the membrane NADH oxidase identifiable on normal, non-diseased cells and is characterized by the fact that it is heat sensitive (loss of activity when subjected to 70 degrees Celsius for 10 min.) and preferentially activated under hypotonic conditions. The term "aberrant" when used in the context of ecto-nox protein or membrane NADH oxidase herein refers to an ecto-nox protein which does not function properly. In particular embodiments, the ecto-nox protein is in a permanently activated state and characterized by the fact that it is heat insensitive (retains activity when subjected to 70 degrees Celsius for 10 min.) and shows highest activity under isotonic conditions.
In particular embodiments, the present invention provides for compositions comprising a MAF, for use in the treatment of CFS/ME and CFS/ME related diseases, which are capable of reducing aberrant ecto-nox functioning in patients suffering from CFS/ME and/or CFS/ME related diseases. Most particularly, the invention provides compositions capable of ensuring a 10% reduction, more particularly a 20%, 30%, 40%, 50%, 60%, 70% or 80% or more reduction of the aberrant ecto-nox functioning in patients with CFS/ME compared to prior to administration of the composition of the invention, more particularly as determined by the methods disclosed in WO2010109009. More particularly, the compositions of the invention can be used to normalize the ecto-nox functioning in a patient suffering from CFS/ME or a CFS/ME related disorder.
In particular embodiments, the present invention provides compositions for use in the treatment of patients suffering from CFS/ME, wherein the patient is diagnosed to have aberrant ecto-nox functioning, more particularly as determined by the methods disclosed in WO2010109009.

Further indications of CFS/ME also include an increased amount of metabolites in physiological fluids such as urine. The metabolite concentration in physiological fluids can be detected and monitored using reducible dyes, which allows monitoring of the disease (WO2010142322, which is hereby incorporated by reference).
In particular embodiments, the present invention provides for compositions comprising a MAF, for use in the treatment of CFS/ME and/or CFS/ME related diseases, which are capable of reducing aberrant metabolite production in patients suffering from CFS/ME and/or CFS/ME related diseases. Most particularly, the invention provides compositions capable of ensuring a 10% reduction, more particularly a 20%, 30%, 40%, 50%, 60%, 70% or 80% or more reduction of the aberrant metabolite production in patients with CFS/ME compared to prior to administration of the composition of the invention, more particularly as determined by the methods disclosed in WO2010142322. More particularly, the compositions of the invention can be used to normalize the ecto-nox functioning in a patient suffering from CFS/ME or a CFS/ME related disorder.
in particular embodiments, the present invention provides compositions for use in the treatment of patients suffering from CFS/ME, wherein the patient is diagnosed with increased metabolite production, more particularly as determined by the methods disclosed in WO2010142322.

While the link between both conditions remains controversial, there is increasing evidence that a significant number of patients suffering from CFS/ME is infected with xenotropic murine leukemia virus-related virus.
The term "xenotropic murine leukemia virus-related virus" or "XMRV" as referred to herein designates an infectious gamma-retrovirus as found in prostate tumors, particularly in prostate tumors of patients homozygous for RNASEL variant, R462Q (e.g., Urisman et al., PLoS Pathog. 2(3): e25, 2006; Dong et al., Proc. Natl. Acad. ScL USA 104(5): 655, 2007 and WO 2006/110589; all of which are incorporated herein by reference in their entirety). The term "xenotropic murine leukemia virus-related virus" or "XMRV" includes any strain of the virus including XMRV VP35 (GenBank Accession No. DQ241301), XMRV VP42 (GenBank Accession No. DQ241302) and XMRV VP62 (GenBank Accession No. DQ399707).
In addition, a number of diseases have been associated with CFS/ME because of the similarity of the symptoms observed with CFS/ME patients and patients suffering from such CFS/ME-related diseases. For instance, fibromyalgia, autoimmune thyroid disease, xenotropic murine leukemia virus-related virus (XMRV) and several other diseases have been suggested as being related to CFS/ME and presenting similar symptoms of widespread pain, fatigue, feeling run down, sluggish muscle cramps and pains, unexplained or excessive weight gain, inability to lose weight, gastrointestinal problems, irritable bowel syndrome, poor sleeping, headaches and migraines, constipation, orthostatism and exhaustion.
Although such CFS/ME-related diseases may contribute in the pathogenesis of CFS/ME, often a causative link between CFS/ME and such diseases has not been unequivocally established.
Accordingly, it is envisaged herein that while a subgroup of patients with CFS/ME may additionally have one or more CFS/ME-related diseases, such as an infection with XMRV not all patients with CFS/ME are necessarily suffering from an additional CFS/ME related disease, such as an infection with XMRV. In addition, not all patients with for example diagnosed to be infected with XMRV, are additionally diagnosed with CFS/ME, despite that fact that they may show at least some symptoms of CFS/ME.
The compositions of the present invention are envisaged to be of use in the treatment of both CFS/ME and CFS/ME related diseases. In particular embodiments however, the present invention relates to the treatment of patients with CFS/ME in which an infection with XMRV has been identified.

The present invention relates to a MAF for use in the treatment of CFS/ME and/or CFS/ME related diseases or disorders, such as an infection by XMRV in a mammal. In particular embodiments, the present invention relates to use of a MAF for the manufacture of a medicament for the treatment of CFS/ME and/or infection by XMRV in a mammal. In particular embodiments, the present invention relates to the a MAF for use in the treatment of CFS/ME and/or infection by XMRV in a mammal.
In particular embodiments, the present invention relates to the use of a MAF in the manufacture of a medicament for the treatment of, CFS/ME and/or infection by XMRV in a mammal.

In particular embodiments, the present invention relates to compositions comprising a MAF for use in the treatment of a patient suffering from CFS/ME and/or one ore more CFS/ME-related diseases, such as an infection by XMRV. In particular embodiments, the patient is also characterized as having aberrant ecto-nox functioning, e.g. by the method disclosed in WO2010109009. In particular embodiments, the patient is also diagnosed with aberrant and/or increased metabolite production, e.g. by the method disclosed in WO2010142322.

In further particular embodiments, the present invention provides in compositions as described above, comprising a pharmaceutically acceptable excipient and a therapeutically effective amount of said MAF. In particular embodiments, the MAF as described herein, is GcMAF.
In particular embodiments, the compositions of the present invention comprises MAF as the sole active ingredient or consists of a MAF. However, treatment regimes are also envisaged wherein the MAF is administered in combination with one or more other therapeutic compounds. The present invention further envisages treatment regimes wherein a patient suffering from CFS/ME or a CFS/ME related disorder is treated with (Gc)MAF in combination with one or more compounds selected from a viral inhibitor and/or an ecto-nox modifying agent,

More particularly, the present invention relates to the therapeutic use of a MAF as described above used in combination with at least one specific inhibitor of a virus wherein said virus is associated with CFS/ME, more particularly wherein said virus is XMRV. In particular embodiments, (one of) said viral inhibitor(s) is Raltegravir^{®}.
In particular embodiments, the present invention relates to the therapeutic use of a MAF as described above used in combination with an ecto-nox modifying agent. The term "ecto-nox protein modifying agent" as used herein refers to any compound capable of converting a normal ecto-nox protein (or membrane NADH oxidase) into an aberrant ecto-nox protein (or membrane NADH oxidase) or capable of inducing aberrant NADH oxidase activity in a sample. In particular embodiments, the ecto-nox protein modifying agent is a molecule having NADH oxidase activity capable of competing with the ecto-nox protein, or a molecule capable of inhibiting the association of an ecto-nox protein with the plasma membrane, or an NADH oxidase inhibitor. In particular embodiments, the ecto-nox modifying agent is selected from the group consisting of a prion molecule (such as NADH oxidase) and a solvent (such as dimethyl sulfoxide, i.e. DMSO).
In further particular embodiments, the present invention relates to the therapeutic use of a MAF as described above used in combination with at least one mammalian liver extract. Mammalian liver extract has shown NADH oxidase activity and an antiviral activity against certain viruses. Mammalian liver extract has been commercialized under the names of Kutapressin^{®} and Nexavir^{®}. US5,055,296 describes the use of a mammalian liver extract for the treatment of viral infections and chronic fatigue syndrome. The extract is described to be thermostable, acetone-insoluble and soluble in water. Chemical analysis of the liver extract revealed at least five polypeptides of which one was found to have bradykinin-potentiating activity. In US5,334,395, which relates to the use of a mammalian liver extract in the treatment of Epstein Barr Virus (EBV) infection, 9 peptides are identified in the Kutapressin extract as having angiotensin converting enzyme inhibitory activity. One of these peptides is demonstrated to be capable of inhibiting EBV infection in vitro.

The inventors further discovered positive synergistic effects when the MAF of the present invention was used in combination with Vitamin B12. In particular embodiments, the present invention thus relates to the therapeutic use of a MAF as described above used in combination with Vitamin B12. In particular embodiments, the MAF of the present invention and Vitamin B12 are administered to the patient separately.

The inventors also discovered positive synergistic effects when the MAF of the present invention was used in combination with one or more compounds selected from interferon alpha, interferon beta, interferon gamma and/or interleukin-7. In particular embodiments, the present invention relates to the therapeutic use of a MAF according to the present invention used in combination with one or more compounds selected from interferon alpha, interferon beta, interferon gamma and/or interleukin-7. In particular embodiments, the MAF of the present invention and interferon alpha, interferon beta, interferon gamma and/or interleukin-7 are administered to the patient separately.

As described hereabove, in the treatment regimens envisaged in the context of the present invention the MAF can be administered separately or simultaneously with another compound of interest. When administered simultaneously, the compounds may be comprised in the same or separate formulations. However, in particular embodiments, the invention provides compositions for combined treatment regimens. Thus, in particular embodiments, the present invention relates to a MAF or composition as described above, and/or to the use thereof,wherein said MAF is provided in combination with at least one other pharmacologically active compound, for example, a viral inhibitor, an ecto-nox protein modifying agent, a mammalian liver extract, vitamin B12, interferon alpha, interferon beta, interferon gamma and/or interleukin-7.
In particular embodiments, at least one other pharmacologically active compound is selected from a viral inhibitor, and an ecto-nox protein modifying agent. In particular embodiments the composition comprises, or further comprises a mammalian liver extract, preferably Nexavir®. In particular embodiments the composition comprises, or further comprises Vitamin B12. In particular embodiments, the composition comprises, or further comprises one or more compounds selected from interferon alpha, interferon beta, interferon gamma and/or interleukin-7.

The present invention provides a use of a MAF or composition as described above in the manufacture of a medicament for the treatment of CFS/ME and/or for inhibiting infection by XMRV in a mammal, wherein said MAF is provided in a conjugate or in a pharmaceutical composition further comprising a pharmaceutically acceptable excipient and a therapeutically effective amount of said MAF.
The term "therapeutically effective amount" as used herein means that amount of MAF, compound, conjugate or pharmaceutical agent that elicits the biological or medicinal response in a tissue, system, animal or human that is being sought by a researcher, veterinarian, medical doctor or other clinician, which includes alleviation of the symptoms of the disease being treated.

The pharmaceutical composition can be prepared in a manner known per se to one of skill in the art. For this purpose, at least one MAF, one or more solid or liquid pharmaceutical excipients and, if desired, in combination with at least one other pharmaceutical active compound, are brought into a suitable administration form or dosage form which can then be used as a pharmaceutical in human medicine or veterinary medicine.
Particular forms of the pharmaceutical composition may be, for example, solutions, suspensions, emulsions, creams, tablets, pills, capsules, nasal sprays, liposomes or micro-reservoirs, especially compositions in orally ingestible or sterile injectable form, for example, as sterile injectable aqueous or oleaginous suspensions or suppositories and sterile packaged powders (which are usually reconstituted prior to use) for administration as a bolus and/or for continuous administration. The solid carrier may comprise one or more excipients, e.g. lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, fillers, disintegrating agents, binders, e.g. cellulose, carboxymethylcellulose or starch or anti-stick agents, e.g. magnesium stearate, to prevent tablets from adhering to tabletting equipment, (sterile) water, methylcellulose, methyl- and propylhydroxybenzoates, talc, magnesium stearate, edible oils, vegetable oils and mineral oils or suitable mixtures thereof. Tablets, pills and boluses may be formed so as to disintegrate rapidly or to provide slow release of the active ingredient. The formulations can optionally contain other pharmaceutically active substances (which may or may not lead to a synergistic effect with the MAF of the invention) and other substances that are commonly used in pharmaceutical formulations, such as lubricating agents, wetting agents, emulsifying, and suspending agents, dispersing agents, desintegrants, bulking agents, fillers, preserving agents, sweetening agents, flavoring agents, flow regulators, release agents, etc. The compositions may also be formulated so as to provide rapid, sustained, or delayed release of the active compound(s) contained therein, for example using liposomes or hydrophilic polymeric matrices based on natural gels or synthetic polymers.
For the purposes of the present invention, the MAF or compounds or the pharmaceutical composition of the present invention may be administered by any of several routes including but not limited to oral administration, buccal (e.g. sub-lingual) administration, topical administration and parenterally injection, i.e. including intravenous, intraperitoneal, intramuscular, intrasternal or subcutaneous injection. The MAF or compound of the present invention will generally be administered in an "effective amount", by which is meant any amount of a MAF that, upon suitable administration, is sufficient to achieve the desired therapeutic or prophylactic effect in the individual to which it is administered.
Usually, depending on the condition to be prevented or treated and the route of administration, such an effective amount will usually be between 0.01 to 100 ng, preferably between 0.1 and 50 ng and even more preferably between 0.1 and 10 ng of the MAF per kilogram body weight, which may be administered as a single daily dose, divided over one or more daily doses, or essentially continuously, e.g. using a drip infusion.
In particular embodiments, the effective amount is between 25 and 200 ng MAF/dose. In further particular embodiments, the effective amount is between 25-500ng, more particularly about 10 ng MAF per week.
In particular embodiments, the MAF or pharmaceutical composition of the present invention are administered once per week, during 2 to 50 weeks, preferably during 5 to 40 weeks, such as during 15 weeks.
The amount(s) to be administered, the route of administration and the further treatment regimen may be determined by the treating clinician, depending on factors such as the age, gender and general condition of the patient and the nature and severity of the disease/symptoms to be treated.
In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.
For an oral administration form, the compositions of the present invention can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcolholic solutions are water, glycerol and sugar solutions. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.
The oral administration of a pharmaceutical composition comprising the MAF according to the invention, is suitably accomplished by uniformly and intimately blending together a suitable amount of said MAF in the form of a powder, optionally also including a finely divided solid carrier, and encapsulating the blend in, for example, a hard gelatin capsule. The solid carrier can include one or more substances, which act as binders, lubricants, disintegrating agents, coloring agents, and the like. Suitable solid carriers include, for example, calcium phosphate, magnesium stearate, talc, sugars, lactose, dextrin, starch, gelatin, cellulose, polyvinylpyrrolidine, low melting waxes and ion exchange resins.
Oral administration of a pharmaceutical composition comprising the MAF according to the present invention can also be accomplished by preparing capsules or tablets containing the desired amount of said MAF, optionally blended with a solid carrier as described above. Compressed tablets containing the pharmaceutical composition of the invention can be prepared by uniformly and intimately mixing the active ingredient with a solid carrier such as described above to provide a mixture having the necessary compression properties, and then compressing the mixture in a suitable machine to the shape and size desired. Molded tablets maybe made by molding in a suitable machine, a mixture of powdered compound or MAF according to the invention moistened with an inert liquid diluent.
For parenterally administration such as subcutaneous or intravenous administration, the MAF or compounds of the present invention, if desired with the substances customary therefore such as solubilizers, emulsifiers or further auxiliaries, are brought into solution, suspension, or emulsion. The MAF or compounds of the invention can also be lyophilized and the lyophilizates obtained used, for example, for the production of injection or infusion preparations. Suitable solvents are, for example, water, physiological saline solution or alcohols, e.g. glycerol, in addition also sugar solutions such as glucose or mannitol solutions, or alternatively mixtures of the various solvents mentioned. The injectable solutions or suspensions may be formulated according to known art, using suitable nontoxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.
For topical administration, which includes the application of medicinal substances to the skin or various body orifices, the MAF or compounds of the present invention may be applied in a variety of forms such as liquid, semisolid or solid. Some of the possible components of topical, transdermal and transmucosal formulations and delivery devices include solubilizing agents, suspending agents, dispersing agents, preservatives, animal and vegetable fats, oils, or waxes, emollients, stabilizing agents, thickening or gelling agents, buffering agents, adhesive agents, adjuvants and additives, emulsifiers and penetration enhancing agents as known to the person skilled in the art.
For sub-lingual administration, the MAF or compounds of the present invention may be formulated in admixture with with at least a proper excipient typical of sublingual formulations. As non limiting example for the artisan skilled in the art, said excipients are selected from the group including: water-soluble inert excipients (such as, for example, mannitol, sorbitol, lactose); water-insoluble excipients which help the delivery of the active substance at the sublingual mucosa level (such as, for example, microcrystalline cellulose); sweeteners (such as, for example, aspartame, sodium saccharate); flavourings (such as, for example, peach, apricot, banana, strawberry, orange, mandarin flavours); lubricants (such as, for example, magnesium stearate, PEG 6000); taste correctors (such as, for example, sodium citrate); other excipients, additives, carriers commonly used in the formulation pharmaceutical art.

In addition to administration with conventional carriers, the MAF or compounds of the present invention may be administered by a variety of specialized oligonucleotide or nucleic acid delivery techniques such as by encapsulation in various encapsulating materials, such as in unilamellar liposomes (Bayard et al., Eur. J. Biochem. 151: 319, 1985) or by conjugation to carrier molecules such as poly(L-lysine). Reconstituted Sendai virus envelopes have been successfully used to deliver RNA and DNA to cells (Arad et al., Biochem. Biophys. Acta. 85: 88, 1986). Moreover, the virus envelope is not limited to Sendai virus, but could include encapsulation in any retroviral amphotrophic particle. These techniques may be utilized for introduction of the present Macrophage Activation Factors into cells.

The pharmaceutical compositions of this invention can be administered to humans in dosage ranges specific for each MAF or compound comprised in said compositions. The MAF or compounds comprised in said composition can be administered together or separately.

It will be understood, however, that specific dose level and frequency of dosage for any particular patient may be varied and will depend upon a variety of factors including the activity of the specific MAF or compound of the invention employed, the metabolic stability and length of action of that MAF or compound, the age, body weight, general health, sex, diet, mode and time of administration, rate of excretion, drug combination, the severity of the particular condition, and the host undergoing therapy.

### EXAMPLES

### 1. Effect of GcMAF on a patient suffering from CFS/ME.

The patient, a 29 year old female, has suffered from CFS/ME ever since an acute Epstein - Barr virus infection at the age of 17. The patient also tested positive for XMRV in three laboratories with different diagnostic techniques. The patient was treated with intravenous injections of GcMAF, once a week during 10 weeks. Upon each injection, 200 ng of GcMAF was administered to the patient.
The patient showed significant clinical improvement after the treatment with GcMAF. In addition, indicators of NK cell activity and inflammation were positively affected. The results are summarized in Table 1.
The GcMAF treatment resulted in a decrease of Interleukin-8 (IL8) concentration in the serum. IL8 is a protein produced by macrophages and other cell types such as epithelial cells, and is associated with inflammation. Thus, the decrease of IL8 concentration indicates reduced inflammation.
Moreover, also a significant decrease of the patient's C4a serum level was measured after treatment with GcMAF. The C4a measurement was performed using a Becton Dickinson OptEIA human C4a elisa kit.

Additionally, an increase of intracellular perforin in PBMCs was observed. The Perforin mRNA level was measured by real-time PCR, using gene expression assay reagents from Applied Biosystems.

As a conclusion, the results in Table 1 show that the treatment with GcMAF on a patient suffering from CFS/ME resulted in increased NK cell activity and reduced inflammation. As reduced NK cell activity and inflammation are associated with CFS/ME, this indicates that GcMAF treatment is effective in the treatment of patients suffering from CFS/ME.

**Table 1: Laboratory results before and after GcMAF treatment.**

| | Before | After |
|---|---|---|
| IL8 serum (pg/mL) | 29 | 12.6 |

| Perforin PBMC | 552 | 1184 |
|---|---|---|
| C4a serum level (ng/mL) | 4519 | 2292 |

### 2. Clinical study on 108 patients suffering from CFS/ME.

A clinical test fo the effects of GcMAF was run on 108 CFS/ME patients, aged between 18 and 65. The patients tested positive on Murine Leukemia Virus infection (34 patients), XMRVc (XMRV detection in co-culture - 54 patients) or serology (20 patients). The patients were treated with intravenous injections of GcMAF, once a week. Upon each injection, 25-100 ng GcMAF in 1 mL physiological serum was administered. 75% of the patients were treated with 100 ng GcMAF per week.
Already after fifteen weeks, 68 of the 108 patients showed clear improvements, as listed in Table 2. These results clearly show that the administration of GcMAF results in clinical improvements of patients suffering from CFS/ME.

**Table 2: Clinical improvements in 68 after 15 weeks of GcMAF treatment.**

| **Clinical improvement** | **Fraction of patients** |
|---|---|
| Fatigue reduced or disappeared | 44/68 |
| Improvement of sleep quality | 39/68 |
| Pain reduced or disappeared | 35/68 |
| Memory/concentration improved | 27/68 |
| Faster or normalized recovery | 42/68 |
| Orthostatism reduced or disappeared | 22/68* |
| Digestive problems reduced or disappeared | 22/68 |

| | |
|---|---|
| * Not all patients suffered from orthostatism prior to GcMAF treatment. | |

## Claims

1. A composition comprising a Macrophage Activating Factor for use in the treatment of a patient suffering from chronic fatigue syndrome/myalgic encephalomyelitis (CFS/ME) and/or an infection by xenotropic murine leukemia virus-related virus (XMRV).

2. The composition according to claim 1, wherein said patient is **characterized by** reduced NK cell activity and increased inflammatory mediators.

3. The composition according to claim 1 or 2, for reducing at least one symptom chosen from the group consisting of substantial impairment in short-term memory or concentration, sore throat, tender lymph nodes, muscle pain, multi-joint pain without swelling or redness, headaches of a new type, pattern, or severity; unrefreshing sleep, post-exertional malaise lasting more than 24 hours, widespread pain, fatigue, feeling run down, sluggish muscle cramps and pains, unexplained or excessive weight gain, inability to lose weight, gastrointestinal problems, irritable bowel syndrome, poor sleeping, headaches and migraines, constipation, orthostatism and exhaustion.

4. The composition according to any one of claims 1 to 3, wherein said patient is diagnosed with aberrant and/or increased metabolite production.

5. The composition according to claims 1 to 4, wherein said Macrophage Activating Factor is GcMAF.

6. The composition according to any one of claims 1 to 5, in which said Macrophage Activating Factor is the only active ingredient.

7. The composition according to any one of claims 1 to 5, further comprising one or more other active ingredients.

8. The composition according to claim 7, where one of said one or more other active ingredients is Vitamin B12.

9. The composition according to claim 7 or 8, where at least one of said one or more other active ingredients is selected from the group consisting of interferon alpha, interferon beta, interferon gamma and interleukin-7.

10. The composition according to any one of claims 7, 8 or 9, wherein at least one of said one or more other active ingredients is selected from a viral inhibitor and/or an ecto-nox-modifying agent.

11. The composition according to any one of claims 7 to 10, further comprising a mammalian liver extract.

12. The composition according to any one of claims 1 to 11, wherein said composition is an enteral composition.

13. The composition according to any one of claims 1 to 12, wherein said patient is diagnosed with CFS/ME and with XMRV infection.
